# EUROPEAN PATENT SPECIFICATION

(11) **EP 1 720 625 B1**
(45) Date of publication and mention of the grant of the patent: **16.11.2011**
(21) Application number: 05707432.0
(22) Date of filing: 16.02.2005
(51) Int. Cl.: B01D 17/02, B01D 61/14, A23L 1/09, A23J 1/16, C07H 1/08, C13K 1/08

(54) **FILTRATION UNIT WITH DECANTER**
FILTRATIONSEINHEIT MIT DEKANTER
UNITE DE FILTRATION AVEC DECANTEUR

(30) Priority: 25.02.2004 EP 04251036
(43) Date of publication of application: 15.11.2006
(73) Proprietor: CERESTAR HOLDING BV, 4551 LA Sas Van Gent (NL)
(72) Inventor: GOSSEL, Werner, 41239 Mönchengladbach (DE); RICHTER, Klaus-Henner, 40668 Meerbusch (DE); MAIWORM, Michael, 20255 Hamburg (DE)
(74) Representative: Wilkinson, Stephen John
(86) International application number: PCT/EP2005/001563
(87) International publication number: WO 2005/079945

(56) References cited:
- FR-A- 2 760 756
- US-A- 4 978 454
- US-A- 5 173 190
- US-A- 6 068 705
- DATABASE WPI Section Ch, Week 199409 Derwent Publications Ltd., London, GB; Class D17, AN 1994-072961 XP002289552 & RU 2 001 954 C (KARAPUTADZE T M) 30 October 1993 (1993-10-30)
- SINGH N, CHERYAN M: "Performance characteristics of a ceramic membrane system for microfiltration of corn starch hydrolysate" CHEM ENG COMM, vol. 168, 1998, pages 81-95, XP009034254
- DATABASE COMPENDEX ENGINEERING INFORMATION, INC., NEW YORK, NY, US; 31 October 1998 (1998-10-31), SINGH N, CHERYAN M: "Process design and economic analysis of a ceramic membrane system for microfiltration of corn starch hydrolysate" XP002289551 Database accession no. EIX99084520145

## Description

### Technical Field

The current invention relates to an improved filtration unit with a three-phase-decanter. The filtration unit is applied in purification of starch hydrolysates and phospholipids and/or lysophospholipids are obtained, as well.

### Background

It is well-known to use ceramic microfiltration membranes for the clarification of corn starch hydrolysate, as is described in Chem. Eng. Comm. 1998, Vol. 168, p 81-95 by N. Singh.

US 6,068,705 describes the use of a nanofiltration membrane for producing a low DE starch hydrolysate.

However, starch contains a certain amount of non-carbohydrate residues. Upon hydrolysis into starch hydrolysate, e.g. with enzymatic hydrolysis, these non-carbohydrate residues such as proteins, lipids etc, are liberated and have to be separated from the starch hydrolysates.

RU-2001954-C discloses a method and apparatus for the purification of starch hydrolysates by separating a mixture of starch hydrolysate and a liquid hydrocarbon in a three-phase decanter into bottom, middle and upper layer products. The middle layer product is subsequently subjected to filtration.

US-4978454-A discloses a settling tank type decanter in combination with a membrane filtration unit integrated by means subjecting the middle fraction to the filtration and redirecting both products of the filtration to the decanter.

There is a need for having a process that effectively, efficiently removes the non-carbohydrate residues and improves the performance of the known membrane filtration processes.

The current invention provides such a process and a filtration unit for doing so.

### Summary of Invention

The current invention relates to a filtration unit for separating incoming stream which unit comprises a multiple feed and bleed stage membrane filtration battery and a three-phase-decanter which is installed in-between said multiple feed and bleed stage membrane filtration battery wherein the three-phase-decanter separates the incoming stream in three distinct phases: a heavy phase, an intermediate phase and a light phase, wherein the intermediate phase is substantially free of any dry matter from the heavy and/or light phases and wherein the incoming stream into the three-phase-decanter comprises retentate from a membrane filtration stage in the multiple stage membrane filtration battery and the intermediate phase separated from the incoming stream in the decanter is redirected to the multiple feed and bleed stage membrane filtration battery for further filtration.

Said multiple feed and bleed stage battery is a microfiltration battery, nanofiltration and/or an ultrafiltration battery. In a preferred embodiment, the multiple feed and bleed stage filtration battery is a microfiltration battery and the in-between three-phase-decanter is installed between 5% and 90%, preferably between 10% and 85%, more preferably between 15% and 75%, most preferably between 30% and 60%, of the total surface of the membranes in the microfiltration battery.

The microfiltration battery preferably has ceramic membranes. The current invention further relates to a method for improving a membrane filtration process for separating incoming stream which method comprises the following steps:
a) using a multiple feed and bleed stage membrane filtration battery;
b) installing a three-phase-decanter in-between said multiple feed and bleed stage membrane filtration battery and wherein said three-phase-decanter separates the incoming stream into three distinct phases: a heavy phase, an intermediate phase and a light phase, wherein the intermediate phase is substantially free of any dry matter from the heavy and/or light phases and wherein the incoming stream into the three-phase decanter comprises retentate from a membrane filtration stage in the multiple stage membrane filtration battery and the intermediate phase separated from the incoming stream in the decanter is redirected to the multiple feed and bleed stage membrane filtration battery for further filtration.

In the method the multiple feed and bleed stage filtration battery is a microfiltration battery, nanofiltration battery, and/or an ultrafiltration battery.

The current invention relates to a method wherein the multiple feed and bleed stage filtration battery is preferably a microfiltration battery and the in-between three-phase-decanter is installed between 5% and 90%, preferably between 10% and 85%, more preferably between 15% and 75%, most preferably between 30% and 60%, of the total surface of the membranes in the microfiltration battery.

The membranes applied in the microfiltration battery are preferably ceramic membranes having filtration layers with a pore size of from 50 to 800 nm. The ceramic membranes preferably further have 4 mm to 6 mm channels.

Furthermore, the current invention relates to a process for purifying starch hydrolysate by applying the method of the current invention.

The starch hydrolysate is selected from the group consisting of glucose syrups, high dextrose syrups and maltodextrin syrups.

The current invention relates to a process for purifying maltodextrin syrups characterised in that it comprises the following steps:
a) taking a maltodextrin syrup;
b) introducing the maltodextrin syrup, as the incoming stream, in the method according to the current invention or into a filtration unit according to the current invention;
c) collecting the purified maltodextrin fraction; and
d) optionally drying the purified maltodextrin fraction.

The current invention further relates to a process for purifying glucose syrups characterised in that it comprises the following steps:
a) taking a glucose syrup;
b) introducing the glucose syrup, as the incoming stream, in the method according to the current invention or into the filtration unit according to the current invention;
c) collecting the purified glucose fraction; and
d) optionally drying the purified glucose fraction.

The current invention relates to a process for isolating phospholipids and/or lyso-phospholipids from cereal starch hydrolysate by applying the method of the current invention.

Such a process preferably comprises the following steps:
a) taking a cereal starch hydrolysate;
b) introducing the cereal starch hydrolysate, as the incoming stream, into the filtration unit according to the current invention;
c) collecting the fraction which contains phospholipids and/or lyso-phospholipids;
d) optionally drying the phospholipids and/or lyso-phospholipids fraction;
   and
e) optionally treating with organic solvent or mixture of organic solvents to obtain phospholipids and/or lyso-phospholipids of high purity.

### Description of Figure

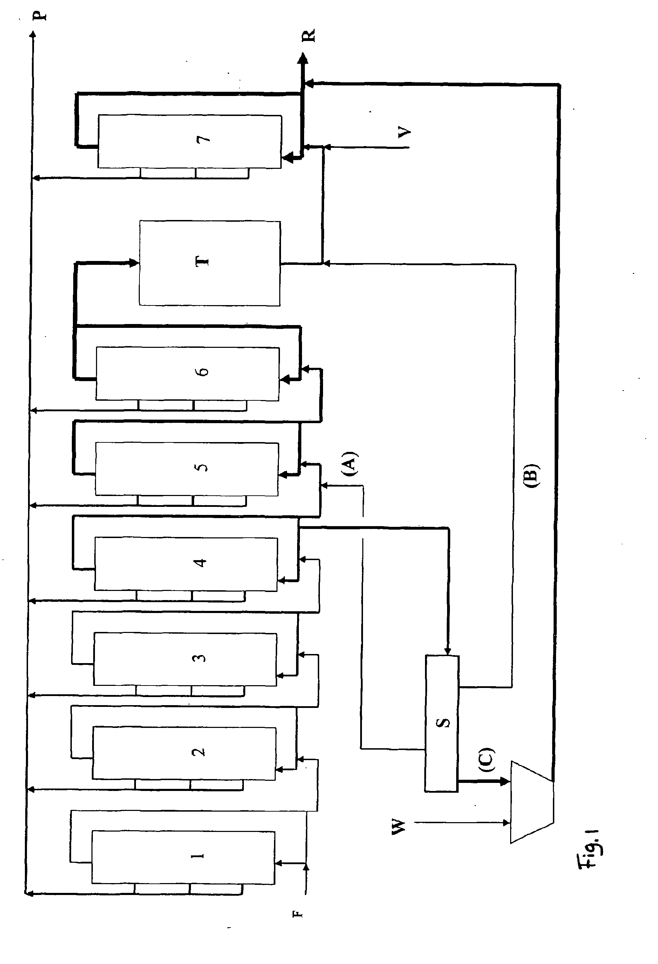

Figure 1 is a schematic presentation of a typical example of the filtration unit of the current invention:
   1-7: 7 stages of the multiple feed and bleed stage microfiltration unit membrane surface of stage 1-3 is 159 m² each membrane surface of stage 4-7 is 118 m² each
   S: three-phase-decanter (Sorticanter™)
   T: Diafiltration Tank 10 m³
   F: Feed stream (incoming stream)
   (A): clarified starch hydrolysate (intermediate phase)
   (C): heavy starch component (dextrins) (heavy phase)
   (B): light proteins, fibres and lipids (phospholipids) (light phase)
   R: Retentate
   P: Permeate
   W: Dilution water

### Detailed description

The current invention relates to a filtration unit according to claim 1 for separating an incoming stream which unit comprises a multiple feed and bleed stage membrane filtration battery and a three-phase-decanter which is installed in-between said multiple feed and bleed stage membrane filtration battery wherein the three-phase-decanter separates the incoming stream in three distinct phases: a heavy phase, an intermediate phase and a light phase, wherein the intermediate phase is substantially free of any dry matter from the heavy and/or light phases and wherein the incoming stream into the three-phase-decanter comprises retentate from a membrane filtration stage in the multiple stage membrane filtration battery and the intermediate phase separated from the incoming stream in the decanter is redirected to the multiple feed and bleed stage membrane filtration battery for further filtration.

The conditions for operating the multiple feed and bleed stage filtration battery can be dialysis, reverse osmosis, ultra-filtration, microfiltration, as appropriate.

The membranes, which can be used, are any solid ceramic, sintered metal or polymeric membranes, which exhibit selectivity for one component of the feed stream over other components of the feed stream.

As starting point of applying a membrane filtration battery, one will generally choose a membrane having a filtration behaviour or a molecular weight cut-off (MCWO), which is often related to membrane pore size that is expected to retain the desired compounds while allowing an undesired compound present in the feed stream (= incoming stream) to pass through the membrane. The desired MWCO or pore size is generally less than the molecular weight of the compound being purified, and is typically greater than the molecular weight of the undesired contaminant that is to be removed from the solution containing the compound being purified.

Nanofiltration membranes generally have a nominal MWCO of between about 150 Da and about 5 kDa, and ultrafiltration membranes generally have a nominal MWCO of between about 1 kDa and about 300 kDa.

Nanofiltration is a pressure driven membrane process with performance characteristics between reverse osmosis and ultrafiltration. Nanofiltration membranes have very good rejection rates of large molecules.

Ultrafiltration is a membrane filtration process operating at 2 to 10 bar pressure and removing very fine particles, micro-organisms and organics as small as 1000 molecular weight.

The performance of the multiple feed and bleed stage filtration battery is, surprisingly, improved by having a three-phase-decanter installed in -between.

The three-phase-decanter separates the incoming stream into three distinct phases: a heavy phase, an intermediate phase and a light phase, wherein the intermediate phase is substantially free of any dry matter from the heavy and/or light phases.

For example, the intermediate phase is not a mixture or emulsion comprising significant amounts of dry matter of the other two phases. Typically, the three distinct phases are aqueous solutions and after applying the filtration unit of the current invention three aqueous phases comprising completely different components are obtained. The three phases have a completely different composition comprising different components. The remainder of any of the other phases in one phase is reduced to a minimum.

The intermediate phase is substantially free of any dry matter present in any of the other two phases, and contains less than 5% w/w, preferably less than 3% w/w, more preferably less than 2% w/w, most preferably less than 1% w/w, of the dry matter of any of the other phases. In a more preferred embodiment, the intermediate phase is substantially free of any dry matter and it contains less than 0.5% w/w, less than 0.4% w/w, dry matter of any of the other phases.

The filtration unit of the current invention is particularly applicable for separating an incoming stream consisting of dry matter A, B and C in aqueous solution into three distinct phases: an aqueous stream containing dry matter A, an aqueous stream containing dry matter B and a third phase which is an aqueous stream containing dry matter C. The intermediate phase with dry matter B is substantially free of the dry matter of any of the other phases, i.e. the intermediate phase is substantially free of dry matter A and/or C.

Said multiple feed and bleed stage filtration battery can be a microfiltration battery, nanofiltration battery, and/or ultrafiltration battery. All of these batteries can be applied and the performance of each of these batteries can be improved by the installation of a three-phase-decanter. A typical example of such a three-phase-decanter is Sorticanter™.

According to the invention, the filtration unit comprises a multiple feed and bleed stage filtration battery and a three-phase-decanter in-between said multiple feed and bleed stage filtration battery. In a preferred embodiment, the multiple feed and bleed stage filtration battery is a microfiltration battery and the in-between three-phase-decanter is installed between 5% and 90%, preferably between 10% and 85%, more preferably between 15% and 75%, most preferably between 30% and 60%, of the total surface of the membranes in the microfiltration battery.

Microfiltration is a membrane filtration process operating at 0.2 - 4 bar pressure allowing molecules of the size of salts, sugars and proteins to pass through the membrane pores, while molecules of the size of bacteria and fat globules are rejected.

A second parameter that is considered in choosing an appropriate membrane for a particular separation is the type of the membrane. The membranes used in each zone are made of conventional membrane material whether inorganic, organic, or mixed inorganic and organic. Typical inorganic materials include glasses, ceramics, cermets, metals and the like.

Thus membranes made of regenerated cellulose, cellulose esters, cellulose ethers, mixtures of cellulose esters and ethers, polyamide, polysulfane, polyurea, polyurethane, polyurea/urethane, ceramics can be used.

In a typical embodiment ceramic membranes are applied. In a more preferred embodiment these membranes have filtration layers with a pore size of from 50 to 800 nm.

Furthermore, in a still more preferred embodiment these ceramic membranes have 4 mm to 6 mm channels.

The current invention further relates to a method for improving a membrane filtration process according to claim 4 for separating an incoming stream, said method comprising the following steps:
a) using a multiple feed and bleed stage membrane filtration battery;
b) installing a three-phase-decanter in-between said multiple feed and bleed stage membrane filtration battery, wherein said three-phase-decanter separates the incoming stream into three distinct phases: a heavy phase, an intermediate phase and a light phase, wherein the intermediate phase is substantially free of any dry matter from the heavy and/or light phases and wherein the incoming stream into the three-phase decanter comprises retentate from a membrane filtration stage in the multiple stage membrane filtration battery and the intermediate phase separated from the incoming stream in the decanter is redirected to the multiple feed and bleed stage membrane filtration battery for further filtration.

Furthermore, the current invention relates to a process for purifying starch hydrolysate by applying the method of the current invention.

Starch can be from any sources.

The preferred starch hydrolysate comprises a member selected from the group consisting of corn starch hydrolysate, wheat starch hydrolysate, root starch hydrolysate, waxy maize starch hydrolysate, mixture thereof, and the like.

The hydrolysate is prepared according to the methods known in the art. In a typical embodiment the starch slurry is liquefied in presence of suitable enzymes such as amylases, and followed by a saccharification step in presence of suitable enzymes such as glucoamylase.

More typical examples are wheat and corn but the current invention is not limited to these mentioned types of starches. Thus, when the starch might still contain non-carbohydrate residues, the filtration unit and/or the method of the current invention is applicable. Furthermore, the starch hydrolysates typically contain higher oligomers (e.g. dextrins, etc.), the so-called heavy starch phase and this is equally well separated from the starch hydrolysate by applying the process of the current invention.

The starch hydrolysate is selected from the group consisting of glucose syrups, high dextrose syrups and maltodextrins syrups. Conventionally, hydrolysates with DE up to 20 are termed maltodextrins, and those with DE values greater than 20 as glucose syrups. A high dextrose syrup is containing at least 90% w/w, preferably at least 92% w/w dextrose, more preferably at least 95% w/w dextrose.

Cereal starches, such as corn- and wheat starch, contain a certain amount of non-carbohydrate residue, which is the so called "mud". In a specific embodiment the mud fraction contains more or less 2/3 lipids and 1/3 proteins based upon dry weight of mud fraction. After the enzymatic hydrolysis of starch into starch hydrolysate, this mud fraction is liberated and has to be separated from the carbohydrate containing fraction. The starch hydrolysate can be an intermediate for further production steps, and therefore it needs to be purified from the so-called mud fraction.

If a carbohydrate is to be purified from a mixture that contains proteins, it is often desirable to remove the proteins as a first step of the purification procedure.

For a carbohydrate that is smaller than the proteins, this separation is accomplished by choosing a membrane that has a porous size or a MWCO which is less than the molecular mass of the protein or other macromolecule to be removed from the carbohydrate containing solution.

Proteins and other macromolecules that have a molecular mass greater than the pore size or MWCO will thus be rejected by the membrane, while the carbohydrate will pass through the membrane.

Conversely, if an oligosaccharide is to be purified from proteins that are smaller than the oligosaccharide, a membrane is used that has a pore size or a MWCO that is larger than the molecular mass of the protein but smaller than that of the oligosaccharide.

A multiple feed and bleed stage microfiltration unit can separate in several sequential stages the mud or retentate fraction from the starch hydrolysate.

The existence of three distinct phases is demonstrated by applying centrifugation trials to the feed stream (incoming stream) of the filtration unit of the current invention.

Surprisingly, a three-phase-decanter as being part of the filtration unit of the current invention is able to separate the three distinct phases.

Besides the starch hydrolysate (the intermediate phase), a white fraction is determined as comprising mainly dextrins (= heavy phase), and a third fraction is containing proteins and lipids (= light phase). None of these phases is a mixture or emulsion of the other two phases. Three distinct compositions are collected.

The light phase contains different Free Fatty Acids (FFA), phospholipids, lyso-phospholipids and proteins.

In a preferred embodiment, the starch hydrolysate is purified by applying it to the filtration unit of the current invention wherein the three-phase-decanter is installed in-between said multiple feed and bleed stage membrane filtration battery and characterised in that said three-phase-decanter separates the incoming stream into three distinct phases, being a heavy starch phase, a light phase containing protein, fibre and phospholipids and the intermediate phase contains the purified, clarified starch hydrolysate. The intermediate phase is substantially free of dextrins, proteins, fibre and phospholipids.

In an even more preferred embodiment, the process for purifying the starch hydrolysate comprises applying it to the filtration unit of the current invention wherein the in-between three-phase-decanter is installed between 5% and 90%, preferably between 10% and 85%, more preferably between 15% and 75%, most preferably between 30% and 60%, of the total surface of the membranes in the multiple feed and bleed stage membrane filtration battery.

The current invention relates to a process for purifying a maltodextrin syrup characterised in that it comprises the following steps:
a) taking a maltodextrin syrup;
b) introducing the maltodextrin syrup, as the incoming stream, in the method according to the current invention or into a filtration unit according to the current invention;
c) collecting the purified maltodextrin fraction; and
d) optionally drying the purified maltodextrin fraction.

The result of applying this process is a split-up into three distinct phases: a) a heavy phase containing starch, dextrins b) the intermediate phase being the purified, clarified maltodextrin, and c) the light phase containing protein, fibre and phospholipids. In particular, the purified maltodextrin stream contains less than 5% w/w, preferably less than 3% w/w, more preferably less than 2% w/w, most preferably less than 1% w/w, even more preferably less than 0.5% w/w of dry matter of the heavy and/or light phases (i.e. the dry matter of the heavy phase: starch stream and/or the light phase: protein, fibre and phospholipids).

The current invention further relates to a process for purifying a glucose syrup characterised in that it comprises the following steps:
a) taking a glucose syrup;
b) introducing the glucose syrup, as the incoming stream, in the method according to the current invention or into the filtration unit according to the current invention;
c) collecting the purified glucose fraction; and
d) optionally drying the purified glucose fraction.

The result of applying this process for purifying the glucose syrups is a split-up into three distinct phases: a) a heavy phase containing starch, dextrins b) the intermediate phase being the purified, clarified glucose syrup, and c) the light phase containing protein, fibre and phospholipids. In particular the purified glucose syrup stream contains less than 5% w/w, preferably less than 3% w/w, more preferably less than 2% w/w, most preferably less than 1% w/w, even more preferably less than 0.5% w/w of dry matter of the heavy and/or light phases (i.e. the dry matter of the heavy phase: starch stream and/or the light phase: protein, fibre and phospholipids).

In a preferred embodiment, the process for the purification of maltodextrins or glucose syrups makes use of the filtration unit of the current invention wherein the in-between three-phase-decanter is installed between 5% and 90%, preferably between 10% and 85%, more preferably between 15% and 75%, most preferably between 30% and 60%, of the total surface of the membranes in the multiple feed and bleed stage membrane filtration battery. The multiple feed and bleed stage membrane filtration battery is preferably a microfiltration unit (battery). More preferably the three-phase-decanter is installed between 40 to 60% of the total surface of the membranes in the microfiltration unit.

In a typical operation of the three-phase-decanter, the solid particles are separated by centrifugal forces on the drum of the decanter and they are discharged by a screw conveyor which is running on a slightly higher speed than the rotating drum.

In an even more preferred embodiment, the three-phase-decanter is installed after elution of about 50% of the total surface of the membranes available in the microfiltration unit (battery).

The current invention also relates to a process for isolating phospholipids and/or lyso-phospholipids from cereal starch hydrolysate by applying the method of the current invention.

Such a process comprises the following steps:
a) taking a cereal starch hydrolysate;
b) introducing the cereal starch hydrolysate, as the incoming stream, in the method of the current invention or into the filtration unit of the current invention;
c) collecting the fraction containing phospholipids and lyso-phospholipids;
d) optionally drying the phospholipids and/or lyso-phospholipids fraction;
   and
e) optionally treating with organic solvent, or mixtures of organic solvents, to obtain phospholipids and/or lyso-phospholipids of high purity.

The steps (d) and e) are optional and interchangeable.

The phospholipids-containing fraction is taken out of the hydrolysate process and can be used as such in further processes, because the dry substance is very high. In a preferred embodiment the dry substance is higher than 50%, preferably higher than 60% and more preferably higher than 70%.

An optional further step in the process is to dry and/or extract the phospholipids and/or lyso-phospholipids fraction with a suitable organic solvent or a mixture of organic solvents. Suitable organic solvents are chloroform, methanol, and the like. Preferably mixtures thereof are used. This phospholipid fraction contains small amounts of phospholipids and different lyso-phospholipids. In a preferred embodiment, by applying the treatment with the organic solvents, the residual small fraction of the dry matter of the intermediate phase (i.e. the starch hydrolysate), is completely removed.

Through the new way of isolation of phospholipids and/or lyso-phospholipids, the direct isolation and production of 1-acyl-lyso-phosphatidylcholin and similar phospholipids are directly possible.

The phospholipids fraction, which is the light phase of the current invention, is isolated via a new process and results in a new composition, which is not described before.

They can be used in pharmaceutical and cosmetic applications. They can be used as emulsifiers in pharmaceutical and cosmetic applications. In a typical example the isolated, dried and solvent treated light phase can be applied as a slimming agent.

The current invention makes use of a three-phase-decanter wherein said decanter separates the incoming stream in three distinct phases: a heavy phase, an intermediate phase and a light phase, wherein the intermediate phase is substantially free of any dry matter from the heavy and/or light phases.

The current invention has the following advantages:
- removing 'mud' as relative dry material and turns it into a high-quality product
- improving capacity of multistage membrane battery without increasing the membrane surface of the battery
- reducing the losses of carbohydrate based material
- new composition of phospholipids and/or lyso-phospholipids

The invention is illustrated by the following, non-limiting example:

### Example 1:

A corn starch slurry (034#4; raw material for starch 03401 Cerestar) at 28-32% ds was hydrolysed with a thermostable α-amylase (0.02-0.08% enzyme on d.s. of corn starch hydrolysate) at 105-110°C, and after two hours converted with 0.06% on d.s. glucoamylase to a hydrolysate with an α-D-glucose content of >95% ds. After a reaction time of 70-90 hours the reaction is finished.
80-110 m³/h of this product at 28-32% ds was then purified on a multiple feed and bleed stage microfiltration battery (Membraflow) with 7 stages with a total membrane (ceramic membranes of Mebraflow with a cut-off of 200nm) surface area of 949 m² and a three-phase-decanter (Sorticanter™) was installed within the battery after stage 4, i.e. after membrane surface area of 595 m² (see Figure 1).

During this purification non starch components, proteins, fibers and lipids, are separated from the carbohydrate fraction

The following three phases were obtained:
1) heavy phase = 10kg/h of a heavy starch component containing material at 15% ds (dextrin phase)
2) intermediate phase= 5 m³/h of a middle phase containing the hydrolysate at 28-32% ds and with a α-D-glucose content of > 95% ds and containing 0.4% w/w of dry matter of light phase
3) light phase = 66 kg/h of a light protein, fibers and lipids containing phase at 75% ds.

The intermediate phase is a complete clear phase (no turbidity).

The light phase No. 3 contained mainly protein, Free Fatty Acids (FFA) and lyso-Phospholipids (LPL) and was separated at a dry substance of 75% and was dried under mild conditions at 40°C for storage.

A further extraction with organic solvents such as a mixture of 75% chloroform, 25% methanol gave a light yellow solid lipid material at room temperature, and it contained FFA and - 15-20% phospholipids.

The phospholipid composition and the FFA content of the extract was measured with ³¹P-NMR-Spectroscopy (Nuclear magnetic resonance) and GC/MS FFA- Esters.

The composition is displayed in Table 1.

**Table 1**

| Extract | 16.69 % d.s. |
|---|---|
| | % ds |
| Phosphatidyl-cholin (Lecithin) | 2,83 |
| 2-acyl-lyso-phosphatidyl-cholin | 1,27 |
| 1-acyl-lyso-phosphatidyl-cholin | 9,34 |
| Phosphatidyl-ethanolamin | 0,46 |
| Lyso-phosphatidyl-ethanolamin | 1,24 |
| Lyso-phosphatidyl-säure | 0,12 |
| Rest | 1,43 |
| Phosphor-% | 0,92 |
| | |
| | |
| | |

| FFA Composition | % |
|---|---|
| Palmitic acid | 31,47 |
| Stearic acid | 2,06 |
| Oleic acid | 14,9 |
| Linoleic acid | 49,6 |

## Claims

1. A filtration unit for separating incoming stream (F) which unit comprises a multiple feed and bleed stage membrane filtration battery (1-7) and a three-phase decanter (S) which is installed in-between said multiple feed and bleed stage membrane filtration battery wherein the three-phase decanter separates the incoming stream into three distinct phases: a heavy phase (C), an intermediate phase (A) and a light phase (B), the intermediate phase is substantially free of any dry matter from the heavy and/or light phases and wherein the incoming stream into the three-phase decanter comprises retentate from a membrane filtration stage in the multiple stage membrane filtration battery and the intermediate phase (A) separated from the incoming stream in the decanter redirected to the multiple feed and bleed stage membrane filtration battery for further filtration.

2. A filtration unit according to claim 1, **characterised in that** the multiple feed and bleed stage membrane filtration battery is a microfiltration battery and the in-between three-phase decanter is installed between 5% and 90%, preferably between 10% and 85%, more preferably between 15% and 75%, most preferably between 30% and 60%, of the total surface of the membranes in the microfiltration battery.

3. A filtration unit according to either claim 1 or claim 2, **characterised in that** the multiple feed and bleed stage membrane filtration battery is a microfiltration battery with ceramic membranes.

4. A method for improving a membrane filtration process for separating incoming stream (F) which method comprises the following steps:
a) using a multiple feed and bleed stage membrane filtration battery (1-7);
b) installing a three-phase decanter (S) in-between said multiple feed and bleed stage membrane filtration battery and wherein said three-phase decanter separates the incoming stream into three distinct phases: a heavy phase (C), an intermediate phase (A) and a light phase (B) wherein the intermediate phase is substantially free of any dry matter from the heavy and/or light phases and wherein the incoming stream into the three-phase decanter comprises retentate from a membrane filtration stage in the multiple stage membrane filtration battery and the intermediate phase (A) separated from the incoming stream in the decanter is redirected to the multiple feed and bleed stage membrane filtration battery for further filtration.

5. A method according to claim 4, **characterised in that** the multiple feed and bleed stage membrane filtration battery is a microfiltration battery and the in-between three-phase decanter is installed between 5% and 90%, preferably between 10% and 85%, more preferably between 15% and 75%, most preferably between 30% and 60%, of the total surface of the membranes in the microfiltration battery.

6. A method according to either claim 4 or claim 5, **characterised in that** the multiple feed and bleed stage membrane filtration battery is a microfiltration battery with ceramic membranes.

7. A method according to claim 6, **characterised in that** the ceramic membranes have filtration layers with a pore size of from 50 to 800 nm.

8. A method according to either claim 6 or claim 7, **characterised in that** the ceramic membranes have 4 mm to 6 mm channels.

9. A process for purifying a starch hydrolysate, **characterised in that** the starch hydrolysate is introduced, as the incoming stream, in the filtration unit according to any one of claims 1 to 3 or in the method according to any one of claims 4 to 8.

10. A process according to claim 9, **characterised in that** the starch hydrolysate is selected from the group consisting of glucose syrups, high dextrose syrups, and maltodextrin syrups.

11. A process for purifying a maltodextrin syrup, **characterised in that** it comprises the following steps:
a) taking a maltodextrin syrup;
b) introducing the maltodextrin syrup, as the incoming stream, in the method according to any one of claims 4 to 8 or into the filtration unit according to any one of claims 1 to 3;
c) collecting the purified maltodextrin fraction; and
d) optionally drying the purified maltodextrin fraction.

12. A process for purifying a glucose syrup, **characterised in that** it comprises the following steps:
a) taking a glucose syrup;
b) introducing the glucose syrup, as the incoming stream, in the method according to any one of claims 4 to 8 or into the filtration unit according to any one of claims 1 to 3;
c) collecting the purified glucose fraction; and
d) optionally drying the purified glucose fraction.

13. A process for isolating phospholipids and/or lyso-phospholipids from a cereal starch hydrolysate, **characterised in that** the cereal starch hydrolysate is introduced, as the incoming stream, in the method according to any one of claims 4 to 8.

14. A process according to claim 13, **characterised in that** it comprises the following steps:
a) taking a cereal starch hydrolysate;
b) introducing the cereal starch hydrolysate, as the incoming stream, in the method according to any one of claims 4 to 8 or into the filtration unit according to any one of claims 1 to 3;
c) collecting the fraction containing the phospholipids and/or lyso-phospholipids;
d) optionally drying the phospholipids and/or lyso-phospholipids fraction;
and
e) optionally treating with organic solvent or mixture of organic solvents, to obtain phospholipids and/or lyso-phospholipids of high purity.

## Patentansprüche

1. Filtrationseinheit zur Trennung eines ankommenden Stroms (F), wobei die Einheit eine Membranfiltrationsbatterie mit mehreren Einspeisungs- und Ablassstufen (1 - 7) und einen Dreiphasendekantierapparat (S) umfasst, der inmitten der Membranfiltrationsbatterie mit mehreren Einspeisungs- und Ablassstufen installiert ist, wobei der Dreiphasendekantierapparat den ankommenden Strom in drei einzelne Phasen trennt: eine schwere Phase (C), eine Zwischenphase (A) und eine leichte Phase (B), wobei die Zwischenphase im Wesentlichen frei ist von jeglicher Trockensubstanz aus der schweren und/oder leichten Phase, und wobei der in dem Dreiphasendekantierapparat ankommende Strom ein Retentat aus einer Membranfiltrationsstufe in der Mehrstufen-Membranfiltrationsbatterie umfasst und die von dem ankommenden Strom in dem Dekantierapparat getrennte Zwischenphase (A) zur weiteren Filtration wieder in die Membranfiltrationsbatterie mit mehreren Einspeisungs- und Ablassstufen geleitet wird.

2. Filtrationseinheit nach Anspruch 1, **dadurch gekennzeichnet, dass** die Membranfiltrationsbatterie mit mehreren Einspeisungs- und Ablassstufen eine Mikrofiltrationsbatterie ist und der Zwischen-Dreiphasendekantierapparat zwischen 5 % und 90 %, vorzugsweise zwischen 10 % und 85 %, stärker bevorzugt zwischen 15 % und 75 %, am stärksten bevorzugt zwischen 30 % und 60 % der Gesamtfläche der Membranen in der Mikrofiltrationsbatterie installiert ist.

3. Filtrationseinheit nach entweder Anspruch 1 oder Anspruch 2, **dadurch gekennzeichnet, dass** die Membranfiltrationsbatterie mit mehreren Einspeisungs- und Ablassstufen eine Mikrofiltrationsbatterie mit Keramikmembranen ist.

4. Verfahren zur Verbesserung eines Membranfiltrationsverfahrens zur Trennung eines ankommenden Stroms (F), wobei das Verfahren die folgenden Schritte umfasst:
a) Verwenden einer Membranfiltrationsbatterie mit mehreren Einspeisungs- und Ablassstufen (1 - 7),
b) Installieren eines Dreiphasendekantierapparates (S) inmitten der Membranfiltrationsbatterie mit mehreren Einspeisungs- und Ablassstufen und wobei der Dreiphasendekantierapparat den ankommenden Strom in drei einzelne Phasen trennt: eine schwere Phase (C), eine Zwischenphase (A) und eine leichte Phase (B), wobei die Zwischenphase im Wesentlichen frei ist von jeglicher Trockensubstanz aus der schweren und/oder leichten Phase, und wobei der in dem Dreiphasendekantierapparat ankommende Strom ein Retentat aus einer Membranfiltrationsstufe in der Mehrstufen-Membranfiltrationsbatterie umfasst und die von dem ankommenden Strom in dem Dekantierapparat getrennte Zwischenphase (A) zur weiteren Filtration wieder in die Membranfiltrationsbatterie mit mehreren Einspeisungs- und Ablassstufen geleitet wird.

5. Verfahren nach Anspruch 4, **dadurch gekennzeichnet, dass** die Membranfiltrationsbatterie mit mehreren Einspeisungs- und Ablassstufen eine Mikrofiltrationsbatterie ist und der Zwischen-Dreiphasendekantierapparat zwischen 5 % und 90 %, vorzugsweise zwischen 10 % und 85 %, stärker bevorzugt zwischen 15 % und 75 %, am stärksten bevorzugt zwischen 30 % und 60 % der Gesamtfläche der Membranen in der Mikrofiltrationsbatterie installiert ist.

6. Verfahren nach entweder Anspruch 4 oder Anspruch 5, **dadurch gekennzeichnet, dass** die Membranfiltrationsbatterie mit mehreren Einspeisungs- und Ablassstufen eine Mikrofiltrationsbatterie mit Keramikmembranen ist.

7. Verfahren nach Anspruch 6, **dadurch gekennzeichnet, dass** die Keramikmembranen Filtrationsschichten mit einer Porengröße von 50 bis 800 nm aufweisen.

8. Verfahren nach entweder Anspruch 6 oder Anspruch 7, **dadurch gekennzeichnet, dass** die Keramikmembranen Kanäle von 4 mm bis 6 mm aufweisen.

9. Verfahren zur Reinigung eines Stärkehydrolysats, **dadurch gekennzeichnet, dass** das Stärkehydrolysat als der ankommende Strom in die Filtrationseinheit nach einem der Ansprüche 1 bis 3 oder in das Verfahren nach einem der Ansprüche 4 bis 8 eingeführt wird.

10. Verfahren nach Anspruch 9, **dadurch gekennzeichnet, dass** das Stärkehydrolysat aus der Gruppe ausgewählt ist, bestehend aus Glucosesirupen, Sirupen mit hohem Dextrosegehalt und Maltodextrinsirupen.

11. Verfahren zur Reinigung eines Maltodextrinsirups, **dadurch gekennzeichnet, dass** es die folgenden Schritte umfasst:
a) Nehmen eines Maltodextrinsirups,
b) Einführen des Maltodextrinsirups als der ankommende Strom in das Verfahren nach einem der Ansprüche 4 bis 8 oder in die Filtrationseinheit nach einem der Ansprüche 1 bis 3,
c) Sammeln der gereinigten Maltodextrinfraktion und
d) gegebenenfalls Trocknen der gereinigten Maltodextrinfraktion.

12. Verfahren zur Reinigung eines Glucosesirups, **dadurch gekennzeichnet, dass** es die folgenden Schritte umfasst:
a) Nehmen eines Glucosesirups,
b) Einführen des Glucosesirups als der ankommende Strom in das Verfahren nach einem der Ansprüche 4 bis 8 oder in die Filtrationseinheit nach einem der Ansprüche 1 bis 3,
c) Sammeln der gereinigten Glucosesirupfraktion und
d) gegebenenfalls Trocknen der gereinigten Glucosefraktion.

13. Verfahren zur Isolation von Phospholipiden und/oder Lysophospholipiden aus einem Getreidestärkehydrolysat, **dadurch gekennzeichnet, dass** das Getreidestärkehydrolysat als der ankommende Strom in das Verfahren nach einem der Ansprüche 4 bis 8 eingeführt wird.

14. Verfahren nach Anspruch 13, **dadurch gekennzeichnet, dass** es die folgenden Schritte umfasst:
a) Nehmen eines Getreidestärkehydrolysats,
b) Einführen des Getreidestärkehydrolysats als der ankommende Strom in das Verfahren nach einem der Ansprüche 4 bis 8 oder in die Filtrationseinheit nach einem der Ansprüche 1 bis 3,
c) Sammeln der Fraktion, enthaltend die Phospholipide und/oder Lysophospholipide,
d) gegebenenfalls Trocknen der Phospholipid- und/oder Lysophospholipidfraktion und
e) gegebenenfalls Behandeln mit organischem Lösungsmittel oder einem Gemisch aus organischen Lösungsmitteln unter Erhalt von Phospholipiden und/oder Lysophospholipiden hoher Reinheit.

## Revendications

1. Unité de filtration pour séparer un courant entrant (F), ladite unité comprenant une batterie de filtration à membrane du type à étages multiples pour un procédé en continu (1-7) et un décanteur triphasé (S) qui est monté en position intermédiaire dans ladite batterie de filtration à membrane du type à étages multiples pour un procédé en continu, le décanteur triphasé séparant le courant entrant en trois phases distinctes :
une phase lourde (C), une phase intermédiaire (A) et une phase légère (B), la phase intermédiaire étant essentiellement exempte de toute matière sèche provenant des phases lourde et/ou légère, et le courant entrant dans le décanteur triphasé comprenant un rétentat provenant d'un étage de filtration à membrane dans la batterie de filtration à membrane du type à étages multiples, et la phase intermédiaire (A) séparée du courant entrant dans le décanteur étant renvoyée à la batterie de filtration à membrane du type à étages multiples pour un procédé en continu, à des fins de filtration ultérieure.

2. Unité de filtration selon la revendication 1, **caractérisée en ce que** la batterie de filtration à membrane du type à étages multiples pour un procédé en continu est une batterie de microfiltration et le décanteur triphasé intermédiaire est monté à concurrence d'une valeur entre 5 % et 90 %, de préférence entre 10 % et 85 %, de manière plus préférée entre 15 % et 75 %, de manière de loin préférée entre 30 % et 60 % de la
surface totale des membranes dans la batterie de microfiltration.

3. Unité de filtration selon la revendication 1 ou 2, **caractérisée en ce que** la batterie de filtration à membrane du type à étages multiples pour un procédé en continu est une batterie de microfiltration comportant des membranes en céramique.

4. Procédé pour perfectionner un processus de filtration à membrane pour séparer un courant entrant (F), ledit procédé comprenant les étapes suivantes consistant à :
a) utiliser une batterie de filtration à membrane du type à étages multiples pour un procédé en continu (1-7) ;
b) monter un décanteur triphasé (S) en position intermédiaire dans ladite batterie de filtration à membrane du type à étages multiples pour un procédé en continu, ledit décanteur triphasé séparant le courant entrant en trois phases distinctes : une phase lourde (C), une phase intermédiaire (A) et une phase légère (B), la phase intermédiaire étant essentiellement exempte de toute matière sèche provenant des phases lourde et/ou légère, et le courant entrant dans le décanteur triphasé comprenant un rétentat provenant d'un étage de filtration à membrane dans la batterie de filtration à membrane du type à étages multiples, et la phase intermédiaire (A) séparée du courant entrant dans le décanteur étant renvoyée à la batterie de filtration à membrane du type à étages multiples pour un procédé en continu, à des fins de filtration ultérieure.

5. Procédé selon la revendication 4, **caractérisé en ce que** la batterie de filtration à membrane du type à étages multiples pour un procédé en continu est une batterie de microfiltration et le décanteur triphasé intermédiaire est monté à concurrence d'une valeur entre 5 % et 90 %, de préférence entre 10 % et 85 %, de manière plus préférée entre 15 % et 75 %, de manière de loin préférée entre 30 % et 60 % de la surface totale des membranes dans la batterie de microfiltration.

6. Procédé selon la revendication 4 ou 5, **caractérisé en ce que** la batterie de filtration à membrane du type à étages multiples pour un procédé en continu est une batterie de microfiltration comportant des membranes en céramique.

7. Procédé selon la revendication 6, **caractérisé en ce que** les membranes en céramique possèdent des couches de filtration dont la dimension des pores s'élève de 50 à 800 nm.

8. Procédé selon la revendication 6 ou 7, **caractérisé en ce que** les membranes en céramique possèdent des canaux de 4 mm à 6 mm.

9. Procédé pour purifier un hydrolysat d'amidon, **caractérisé en ce qu'**on introduit l'hydrolysat d'amidon, à titre de courant entrant, dans l'unité de filtration selon l'une quelconque des revendications 1 à 3 ou bien dans le procédé selon l'une quelconque des revendications 4 à 8.

10. Procédé selon la revendication 9, **caractérisé en ce que** l'hydrolysat d'amidon est choisi parmi le groupe constitué par des sirops de glucose, des sirops riches en dextrose et des sirops de maltodextrine.

11. Procédé pour purifier un sirop de maltodextrine, **caractérisé en ce qu'**il comprend les étapes suivantes consistant à :
a) prélever un sirop de maltodextrine ;
b) introduire le sirop de maltodextrine, à titre de courant entrant, dans le procédé selon l'une quelconque des revendications 4 à 8 ou bien dans l'unité de filtration selon l'une quelconque des revendications 1 à 3 ;
c) récolter la fraction purifiée de maltodextrine ; et
d) le cas échéant, sécher la fraction purifiée de maltodextrine.

12. Procédé pour purifier un sirop de glucose, **caractérisé en ce qu'**il comprend les étapes suivantes consistant à :
a) prélever un sirop de glucose ;
b) introduire le sirop de glucose, à titre de courant entrant, dans le procédé selon l'une quelconque des revendications 4 à 8 ou bien dans l'unité de filtration selon l'une quelconque des revendications 1 à 3 ;
c) récolter la fraction purifiée de glucose ; et
d) le cas échéant, sécher la fraction purifiée de glucose.

13. Procédé pour isoler des phospholipides et/ou des lyso-phospholipides à partir d'un hydrolysat d'amidon de céréales, **caractérisé en ce qu'**on introduit l'hydrolysat d'amidon de céréales à titre de courant entrant, dans le procédé selon l'une quelconque des revendications 4 à 8.

14. Procédé selon la revendication 13, **caractérisé en ce qu'**il comprend les étapes suivantes consistant à :
a) prélever un hydrolysat d'amidon de céréales ;
b) introduire l'hydrolysat d'amidon de céréales, à titre de courant entrant, dans le procédé selon l'une quelconque des revendications 4 à 8 ou bien dans l'unité de filtration selon l'une quelconque des revendications 1 à 3 ;
c) récolter la fraction contenant les phospholipides et/ou les lyso-phospholipides ;
d) le cas échéant, sécher la fraction des phospholipides et/ou des lyso-phospholipides ; et
e) le cas échéant, traiter avec un solvant organique ou avec un mélange de solvants organiques, pour obtenir des phospholipides et/ou des lyso-phospholipides de pureté élevée.
